# EUROPEAN PATENT APPLICATION

(11) **EP 1 674 098 A1**
(43) Date of publication of application: **28.06.2006**
(21) Application number: 04090516.8
(22) Date of filing: 23.12.2004
(51) Int. Cl.: A61K 31/429, A61K 47/40, A61P 35/00, A61P 43/00

(54) **Stable and tolerable parental formulations of highly reactive organic drug substances with low or no solubility in water**

(71) Applicant: Schering Aktiengesellschaft, 13353 Berlin (DE)
(72) Inventor: Uffrecht, Anka, 13465 Berlin (DE); Thomsen, Jens, 10781 Berlin (DE); Sprenger, Claudia, 12059 Berlin (DE); Reer, Olaf, 13465 Berlin (DE); Sachse, Andreas, 13465 Berlin (DE); Renz, Matthias, 13465 Berlin (DE)

(57) **Abstract**

The present invention concerns pharmaceutical formulations of Epothilones suitable for being administered parenterally, such as intravenously.

## Description

### FIELD OF INVENTION

The present invention concerns pharmaceutical formulations of Epothilones suitable for being administered parenterally, such as intravenously.

### BACKGROUND OF THE INVENTION

Epothilones belong to a natural class of substances with cytotoxic effect and which may be administered parenterally. Unfortunately, the natural substances are not sufficiently stable, either chemically or metabolically, for being developed as pharmaceutical agents. The lactone structure in the Epothilone backbone makes the molecule susceptible to degradation, especially at higher pH values such as above neutral pH. Furthermore, the Epothilones are highly lipophilic substances and practically insoluble in water, which render this class of compounds especially difficult to administer intravenously. Parenterally administered formulations require the Epothilones being completely dissolved in a solvent compatible with physiological fluids, such as blood.

Several efforts have been made in order to make available new Epothilone derivatives, which are both chemically and metabolically stable enough for the development of pharmaceutical agents. Furthermore, the Epothilone derivatives should be superior to natural derivatives in terms of their therapeutic range, their selectivity of action and/or undesirable toxic side effects and/or their active strength. WO 00/66589 describes such superior Epothilones, wherein the carbon atom 6 of the Epothilone depicted on page 1 of WO 00/66589 is provided with an alkenyl, alkinyl or an epoxy radical. However, such additional side-chains make such Epothilones even more lipophilic and poorly wet-table in solvents rendering the formulation of pharmaceuticals as an even greater challenge to the skilled person in the pharmaceutical art.

Several methods for increasing the solubility of sparingly water-soluble drugs so as to produce parenteral compatible formulations have been described. US 6,407,079 (Janssen Pharma) describes injectable formulations, wherein a partially etherified β-cyclodextrin (hydroxyethyl, hydroxypropyl, dihydroxypropyl, methyl or ethyl ethers) is added to a drug that is instable or only sparingly soluble in water. The resulting inclusion compound is more water soluble than the drug itself.

Sulfoalkyl ether cyclodextrins and derivatives thereof for use as solubilising agents for water insoluble drugs are previously described in US 5,376,645 and US 5,134,127.

WO 99/396945 describes Epothilones of type A and B formulated as an infusion concentrate or as a lyophilised composition. Prior to administration, the Epothilone should be dissolved in particular solvents, such as PEG/water mixtures, propyleneglycol/water or ethanol/water. The solubility of the Epothilone increases significantly by applying such solvent mixtures. β-Cyclodextrin or mannitol is added as a bulking excipient.

WO 2004/032866 describes lyophilised compositions comprising Epothilones, preferably Epothilone D together with β-cyclodextrins, including sulfoalkyl ether cyclodextrins.

The production of epothilones, and derivatives is carried out according to the methods that are known to one skilled in the art, as they are described in, for example, DE 19907588, WO 98/25929, WO 99/58534, WO 99/2514, WO 99/67252, WO 99/67253, WO 99/7692, EP 99/4915, WO 00/1333, WO 00/66589, WO 00/49019, WO 00/49020, WO 00/49021, WO 00/71521, WO 00/37473, WO 00/57874, WO 01/92255, WO 01/81342, WO 01/73103, WO 01/64650, WO 01/70716, US 6204388, US 6387927, US 6380394, US 02/52028, US 02/58286, US 02/62030, WO 02/32844, WO 02/30356, WO 02/32844, WO 02/14323, and WO 02/8440. Particularly interesting epothilones of the present invention may be produced according to WO 00/66589.

It is an objective of the present invention to provide a composition comprising an Epothilone derivative and which is intended for being administered parenterally, preferably intravenously. The composition should be stable, at least with respect to the Epothilone, during storage and reconstituted, if necessary, by adding a solvent compatible with blood and suitable for parenteral administration without the need of adding surfactants and organic solvents.

### SUMMARY OF THE INVENTION

The present invention relates to parenteral dosage forms comprising Epothilones and the manufacture thereof in which the final dosage form or the manufacturing process meets the following characteristics:
- Fast dissolution of the Epothilone in a liquid, sufficiently volatile for being evaporated during lyophilization conditions, so as to limit the loss of intact Epothilone during the manufacturing process.
- Lyophilising of the liquid Epothilone composition without loosing intact Epothilone
- Lyophilising cake is sufficiently hard.
- Complete and fast dissolution of the lyophilised cake in a physiologically acceptable solvent so as to form a re-constituted composition ready to be administered or further diluted with a suitable physiologically acceptable solvent.
- Chemically stable lyophilised compositions, at least with respect to the stability of Epothilone.
- Physically stable lyophilised compositions.
- Chemically stable re-constituted solution, at least with respect to the stability of Epothilone.
- Physically stable re-constituted solution.
- High concentration of Epothilone in lyophilised cake and/or re-constituted solution
- High Maximum Tolerated Dose following parenteral administration.

By the present invention is provided lyophilised compositions as well as reconstituted compositions thereof, which as an overall concept comprise an Epothilone or a derivative thereof or mixtures thereof in combination with a cyclodextrin.

Unlike previously described parenterally adminsterable compositions of Epothilones, the compositions of the present invention allow for compositions resulting from reconstitution of the lyophilised cake in simple solvents like water or saline water. Addition of an organic solvent and a glycol is not required in order to get the Epothilone easily dissolved. As mentioned in WO 2004/032866, the reconstitution of lyophilisates requires mixtures of water, ethanol and a glycol. Lyophilisates of WO 99/39694 also require an organic solvent. Obviously, the lyophilisates of the present invention is advantageous to those previously described in that the preparation of the final reconstituted composition only requires water for injection or saline.

In one aspect the invention relates to compositions comprising an Epothilone or mixtures of Epothilones, wherein the Epothilone is provided with a more lipophilic side chain than in the naturally occurring Epothilones. According to the formula I depicted herein, the lipophilic side chain refers to R⁴ and is located at the carbon atom numbered 10 given that the Epothilone is fused with an epoxy ring or located at the carbon atom numbered 7 given that R⁷ and R⁸ are a hydrogen atom or taken together is an additional bond. In WO 00/66589 this carbon atom was numbered 6. The compositions of the invention further comprise a cyclodextrin, preferably a β-cyclodextrin derivative that is etherified with hydroxyalkyl groups and/or sulfoalkyl groups resulting in hydroxyalkylated cyclodextrins or sulfoalkylated cyclodextrins.

It has been found that a general concept of formulating Epothilones suitable for being parenterally administered is enabled by the proper selection of excipients providing the required tonicity, pH, stabilisation of the complex between the cyclodextrin and the Epothilone, chemically and physically stability during lyophilisation, chemically and physically stability of the lyophilised composition. This concept is particularly suitable for Epothilones with very poor wettability properties and/or low water solubility, such as the particular derivatives described by formula I herein. Typical examples on further excipients are a tonicifying agent, a filler, a cryoprotectant, a lyoprotectant and a pH regulating agent. In preferred embodiments of the invention, the further pharmaceutically acceptable excipient is selected from mannitol; sorbitol; xylitol; 2-Amino-2-hydroxymethyl-1,3-propandiol; the acid form or salts of citric acid, acetic acid, histidine, malic acid, phosphoric acid, tartaric acid, succinic acid, MES, HEPES, imidazole, lactic acid, glutaric acid and/or glycylglycine, preferably mannitol and/or trometamol (TRIS).

The inventors have also found that especially useful cyclodextrins apply to sulfoalkyl ether cyclodextrins in that such resulting compositions possess the desired tonicity without the need of adding further fillers or tonicity modifying agents.

Therefore, in a third aspect the invention relates to compositions comprising an Epothilone and a sulfoalkylated cyclodextrin (e. g., a sulfopropylated and a sulfobutylated cyclodextrin).

A still further aspect of the invention relates to a complex between an Epothilone according to formula I described herein and a β-cyclodextrin. The cyclodextrin is preferably a hydroxyalkylated β-cyclodextrin or a sulfoalkylated β-cyclodextrin as mentioned above.

Furthermore, the inventors have provided a method of producing the lyophilised composition of the invention, which over come the initial critical step of dissolving the very hydrophobic Epothilones, namely the initial wetting of the Epothilone in a suitable liquid before the solubilising process can take place afterwards. This process enables fast and complete dissolution of the Epothilone and further allows for the Epothilone to stay in solution, in a sufficient period of time, before the evaporation of solvent takes place. By example, the process of dissolving the Epothilone is much faster than the one described in WO 99/39694, Example 10. In fact the use of the process as described in Example 10 of WO 99/39694 would not work for the Epothilones defined herein. It would take days to get the Epothilones as described herein into solution, during which time hydrolysis would take place. Considerable loss of the Epothilone is therefore resulting.

Accordingly, a still another aspect of the invention relates to the manufacturing of a solid composition, such as a lyophilisate, which may be formed from solutions (hereinafter referred to as "original solutions") comprising an Epothilone as described herein, a cyclodextrin as described herein and optionally at least one further pharmaceutically acceptable excipient as defined herein.

The method for producing a composition of the invention comprises the steps of
a) dissolving an Epothilone as defined herein in an organic solvent, such as an alcohol (preferably ethanol); and
b) dissolving a cyclodextrin as defined herein in aqueous solution, optionally together with at least one further pharmaceutically acceptable ingredient as defined herein, such as mannitol and /or tromethamol; optionally
c) adjusting the pH of the resulting mixture of b) to a pH ranging between 5 and 9, preferably 6 and 8, such as about 7.4 using an inorganic acid, such as hydrochloric acid; and
d) mixing the resulting solvents a) and b) or a) and c); and optionally
e) carrying out sterile filtering of d) to achieve the so-called "original solution"
f) removing the solvent from the "original solution" to provide a solid composition.

### DETAILED DESCRIPTION OF THE INVENTION

By the present invention are provided compositions with sufficient and high solubility of an Epothilone by combining an Epothilone and a cyclodextrin in combination with further adapted excipients or by selecting the cyclodextrin carefully. The compositions are preferably further formulated in a form allowing parenteral administration of the composition, such as in the form of a solid solution, such as lyophilized composition, that is further transformed into a composition resulting from re-constitution of the lyophilisate with a suitable solvent.

The term "formulated in a form for parenteral use" generally refers to injectable compositions. Injectable compositions can be administered intravenously, subcutaneosly or by infusion. In preferred embodiments of the invention, the injectable compositions are for intravenous administration.

The terms "lyophilised composition", "lyophilised cake" and "lyophilisate" are interchangeable terms and are used herein to mean the solid composition resulting from processing a liquid composition, such as a solution comprising the Epothilone dissolved or at least partly dissolved, under conditions including at least one step of cooling the solution into ice, followed by at least one step of vacuum drying.

The terms "lyophilisation" and "freeze-drying" are denoted to mean a process upon where liquid is removed from a dissolved or at least partly dissolved composition under conditions involving at least one step of cooling a liquid composition into ice, followed by vacuum drying.

The term "reconstituted solution" refers to a liquid composition resulting from completely dissolving a solid solution, such as a lyophilisate, in a solvent such as water (water for injection), saline or sterile Ringer's solution. The solvent may include further excipients so as to make the reconstituted composition compatible with physiologically relevant fluids, such as blood. The reconstituted compositions are intended for being ready to be administered parenterally or to be further diluted before use.

By the term "sulfoalkyl ether cyclodextrin" is intended a derivative obtained by introducing an anionic-type substituent, such as a (C₂₋₆ alkylene)-SO₃⁻⁻ anionic substituent onto the cyclodextrin. The sulfoalkyl derivative of cyclodextrin can be a single derivative or a mixture of derivatives. As the cyclodextrin derivatives are functionalised with (C₂₋₆ alkylene)-SO₃⁻⁻ groups, the derivatives are charged species. The sulfoalkyl ether cyclodextrin are either substituted at least at one of the primary hydroxyl groups or they are substituted at both the primary hydroxyl groups and at the 3-positioned hydroxyl group. Substitution at the 2-position is theoretically possible.

Typically the solvent for reconstitution is an aqueous solution comprising 75-100% of water by volume, preferably 85%-100% by volume, more preferably 90-100% by volume, most preferably 95-100% by volume. The solvent may comprise further excipients such as inorganic salts like sodium chloride so as to be compatible with physiologically relevant fluids.

The term "solubility" refers to the solubility of Epothilone in a solvent, such as in the reconstituted composition and/or dissolution of an Epothilone in a solvent during the production process.

According to the invention, an Epothilone needs to be combined with one or more agents that increase the solubility of the Epothilone in water. As a first solubilising agent, a cyclodextrin has been found to improve the solubility of an Epothilone. However, in some cases further excipients may be added to further increase the solubility of the Epothilone or to limit degradation of Epothilone during preparation of the parenteral composition. For example further excipients may be added in order to stabilise the Epothilone during admixing of excipients, during removing of liquid, or during storage or after reconstitution.

Therefore, in one aspect the invention provides a composition comprising an Epothilone, a cyclodextrin and at least one pharmaceutically acceptable excipient selected from a tonicifying agent, a filler, a cryoprotectant, a lyoprotectant and a pH regulator.

In another aspect the invention provides a composition comprising an Epothilone, such as an Epothilone derivative as defined by formula I, and a cyclodextrin, the Epothilone is of formula I, 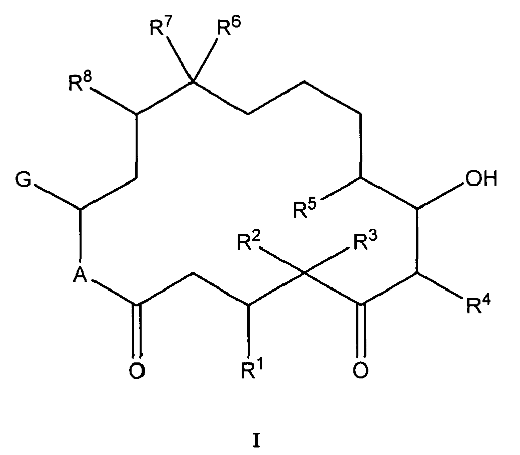 wherein
- R¹: means hydrogen, OR^{1a}, or Halogen, where R^{1a} is hydrogen, SO₂-alkyl, SO₂₋aryl, or SO₂-aralkyl,
- R², R³: are independently C₁-C₁₀ alkyl,
- R⁴: means -(CH₂)ᵣ-C≡C-(CH₂)ₚ-R^{4a}, -(CH₂)ᵣ-CH=CH-(CH₂)ₚ-R^{4a}, 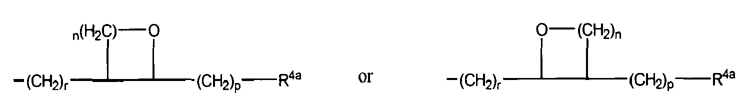
- n: means 0 to 5,
- r: is 0 to 4,
- p: is 0 to 3,
- R^{4a}: means hydrogen, C₁-C₁₀ alkyl, C₆-C₁₂ aryl or C₇-C₂₀ aralkyl, each optionally substituted;C₁-C₁₀ acyl, or, if p > 0, additionally a group OR^{4b}, R^{4b} means hydrogen or a protective group PG;
- R⁵: means C₁-C₁₀ alkyl,
- R⁶: means hydrogen or optionally substituted C₁-C₁₀ alkyl,
- R⁷, R⁸: each mean a hydrogen atom, or taken together an additional bond or taken together an oxygen atom,
- G: means a group X=CR⁹- or a bi- or tricyclic aromatic heterocyclic radical,
- R⁹: means hydrogen, halogen, CN, or a C₁-C₂₀ alkyl,
- X: means a grouping CR¹⁰R¹¹, whereby
R¹⁰, R¹¹ are the same or different and stand for hydrogen, a C₁-C₂₀ alkyl, C₆-C₁₂ aryl, or C₇₋₂₀ aralkyl radical each optionally substituted; or R¹⁰ and R¹¹ together with the methylene carbon atom jointly stand for a 5- to 7-membered carbocyclic ring;
- A: means a group -O- or -NR¹²-,
- R¹²: means hydrogen or C₁-C₁₀ alkyl.

The Epothilone according to formula I may be fused with an epoxide ring in the event where R⁷, R⁸ taken together are an oxygen atom or may exist as one ring system in the event where R⁷, R⁸ each mean a hydrogen atom or taken together mean an additional bond.

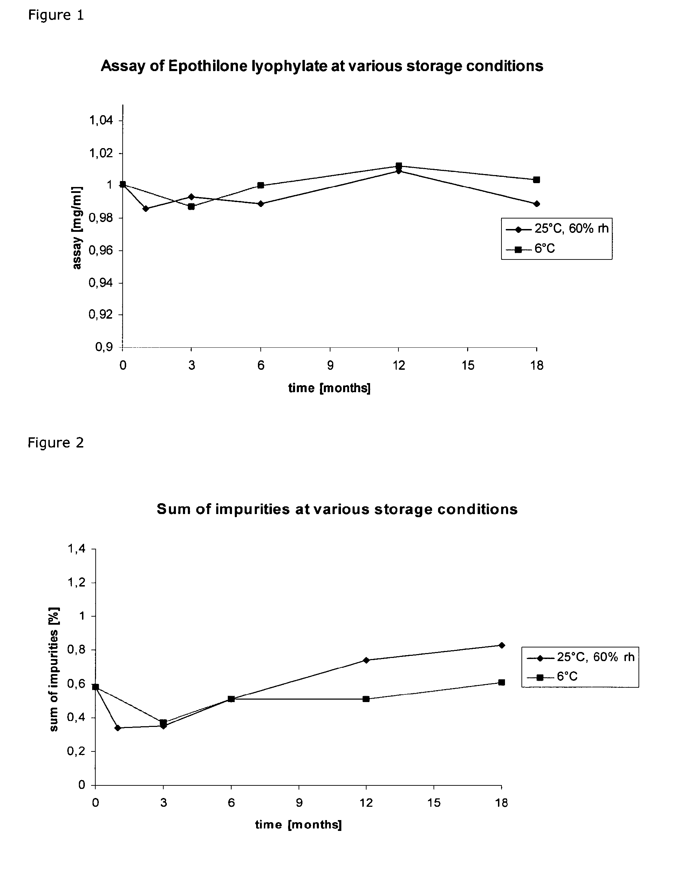

The numbering of the carbon atoms in the Epothilone skeleton will depend on whether the Epothilone is fused with an additional ring, such as an epoxide ring. The formula II as depicted below shows the numbering of carbon atoms in both situations. Figures 1 to 16 refer to the numbering of an Epothilone skeleton, wherein R⁷, R⁸ taken together make an epoxide ring. Figures (1) to (16) refer to the numbering of an Epothilone skeleton, wherein R⁷, R⁸ each mean a hydrogen atom or taken together mean an additional bond.

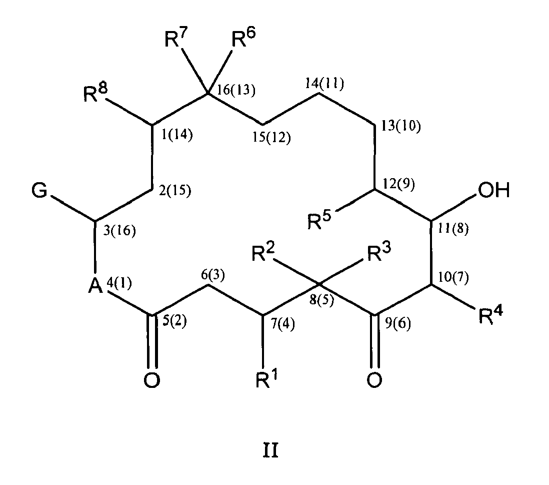

The term "halogen" includes fluorine, chlorine, bromine and iodine.

The term SO₂-alkyl is intended to mean "C₁-C₁₀ alkyl" monosubstituted with -SO₂ group.

The term SO₂-aryl is intended to mean "C₆-C₁₂ aryl" monosubstituted with -SO₂ group.

The term SO₂-aralkyl is intended to mean "C₆-C₁₂ aryl" substituted with one -SO₂ group and with one or two "C₁-C₁₀ alkyl".

The term "C₁-C₁₀ alkyl" is intended to mean a linear or branched saturated hydrocarbon chain wherein the longest chains have from one to ten carbon atoms, such as methyl, ethyl, n-propyl, isopropyl, n-butyl, isobutyl, sec-butyl, tert-butyl, pentyl, isopentyl, neopentyl, hexyl, heptyl, octyl, undecacyl, dodecyl, etc. The C₁-C₁₀ alkyl chain of the present invention may be optionally substituted.

Likewise, the term "C₁-C₂₀ alkyl" is intended to mean a linear or branched saturated hydrocarbon chain wherein the longest chains have from one to twenty carbon atoms.

The term "C₆-C₁₂ aryl " is intended to mean a substituted or unsubstituted carbocyclic aromatic radical or heterocyclic radical consisting of between 6 and 12 carbon atoms. Moreover, the term "aryl" includes fused ring systems wherein at least two aryl rings share at least one chemical bond. Illustrative examples of "aryl" rings include phenyl, naphthalenyl. A preferred aryl group is phenyl and substituted phenyl groups, carrying one or two, same or different, of the substituents listed above. The preferred pattern of substitution is *para* and/or *meta*. Representative examples of aryl groups include, but are not limited to, phenyl, 3-halophenyl, 4-halophenyl, 3-hydroxyphenyl, 4-hydroxyphenyl, 3-aminophenyl, 4-aminophenyl, 3-methylphenyl, 4-methylphenyl, 3-methoxyphenyl, 4-methoxyphenyl, 3-cyanophenyl, 4-cyanophenyl, dimethylphenyl, naphthyl, hydroxynaphthyl, hydroxymethylphenyl, trifluoromethylphenyl, alkoxyphenyl.

The term "aromatic heterocyclic radical" is intended to mean a substituted or unsubstituted aromatic heterocyclic radical consisting of between 6-12 carbon atoms containing one or more heteroatoms. Representative examples of aromatic heterocyclic radicals are furyl, thienyl, pyridyl, pyrazolyl, pyrimidinyl, oxazolyl, pyridazinyl, pyrazinyl, quinolyl, thiazolyl, benzothiazolyl, benzoxazolyl, quinoline which can be substituted in one or more places by halogen, OH, O-alkyl, CO₂H, CO₂-alkyl, NO₂, N₃, CN, C₁-C₁₀ alkyl, C₁-C₁₀ acyl, C₁-C₁₀ acyloxy groups. Heteroatoms in the heteroaryl radicals can be oxidized; thus, for example, the benzothiazole ring can be present in the form of N-oxide. Preferred aromatic heterocyclic radicals include benzothiazolyl, benzoxazol, and quinoline; more preferably C₁₋C₁₀ alkyl substituted benzothiazolyl.

The term "C₇-C₂₀ aralkyl" is intended to mean a carbocyclic aromatic ring or ring system consisting of between 6 and 12 carbon atoms, preferably 6 to 10, and in the alkyl chain 1 to 8, preferably 1 to 4 atoms, wherein at least one aryl ring share at least one chemical bond with a C₁-C₈ alkyl. As aralkyl radicals, for example, benzyl, phenylethyl, naphthylmethyl, naphthylethyl, furylmethyl, thienylethyl, and pyridylpropyl are suitable. The rings can be substituted in one or more places by halogen, OH, O-alkyl, CO₂H, CO₂₋alkyl, NO₂, N₃, CN, C₁-C₁₀ alkyl, C₁-C₁₀ acyl, C₁-C₁₀ acyloxy groups.

The term "C₁-C₁₀ acyl" is intended to mean a linear or branched saturated hydrocarbon chain wherein the longest chains have from one to ten carbon atoms and wherein one of the carbon atom is a C(=O)O radical.

The alkoxy groups that are contained in X in general formula I are in each case to contain 1 to 20 carbon atoms, whereby methoxy, ethoxy, propoxy, isopropoxy and t-butyloxy groups are preferred.

The term "Epothilone" in general is meant to encompass all kinds of substances belonging to the class of Epothilones, naturally or synthetically made, either as a single Epothilone or a mixture of Epothilones. That is to say that the term "Epothilone" refers to any Epothilone, such as Epothilone A, Epothilone B, Epothilone C, Epothilone D, Epothilone E, Epothilone F, analogs, derivatives, salts and mixtures thereof. Preferably, the Epothilone is Epothilone A, Epothilone B, Epothilone C, Epothilone D or derivatives thereof or salts thereof or mixtures thereof. In some particular embodiments, the Epothilone is an Epothilone B derivative according to formula I above.

In some interesting embodiments of the invention, the Epothilone is an Epothilone derivative, wherein the carbon atom 10(7) in the 16-membered macrolide system is provided with an alkenyl, alkinyl or epoxide group as defined above (R⁴ in formula I) instead of the methyl group in the natural occuring Epothilones. Thus, an Epothilone of the present invention refers in general to a derivative of any Epothilone, such as Epothilones A, B, C, D, E or F, in which carbon atom 10(7) of the 16-membered macrolide system is provided with an alkenyl, alkinyl or epoxide group as defined by R⁴ in formula I. As said Epothilones B are of particular interest to the present invention, which means that the above-mentioned derivatives are preferably derivatives of Epothilone B.

In still interesting embodiments of the invention, the Epothilone is an Epothilone derivative selected from:
(1S, 3S (E), 7S, 10R, 11S, 12S, 16R)-7,11-dihydroxy-10-(prop-2-yn-1-yl)-3-(1-methyl-2-(2-methylthiazol-4-yl)ethenyl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzoxazol-5-yl)-8,8,12,16-tetramethyl-4,17-dioxabicyclo [14.1.0] heptadecane-5,9-dione;
(1S, 3S (E), 7S, 10R, 11R, 12S, 16R)-7,11-dihydroxy-10-(prop-2-yn-1-yl)-3-(1-fluoro-2-(2-methyloxazol-4-yl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-chloro-2-(2-methylthiazol-4-yl)ethenyl)-1-oxa-5,5,9,13-tetramethyl-7-(prop-2-en-1-yl)-cyclohexadec-13-ene-2,6-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(4S, 7R, 8S, 9S, 13E/Z, 16S (E))-4,8-dihydroxy-16-(1-methyl-2-(2-pyridyl)ethenyl)-1-oxa-5,5,9,13-tetramethyl-7-(but-3-yn-1-yl)-cyclohexadec-13-ene-2,6-dione;
(4S, 7R, 8S, 9S, 13E/Z, 16S (E))-4,8-dihydroxy-16-(1-methyl-2-(2-pyridyl)ethenyl)-1-oxa-5,5,9,13-tetramethyl-7-(but-3-en-1-yl)-cyclohexadec-13-ene-2,6-dione;
(1S, 3S (E), 7S, 10R, 11S, 12S, 16R)-7,11-dihydroxy-10-(prop-2-en-1-yl)-3-(1-methyl-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-methyl-2-(2-pyridyl)ethenyl)-I-oxa-5,5,9,13-tetramethyl-7-(prop-2-yn-1-yl)-cyclohexadec-13-ene-2,6-dione;
(4S, 7R, 8S, 9S, 13E/Z, 16S (E))-4,8-dihydroxy-16-(1-fluoro-2-(2-methylthiazol-4-yl)ethenyl)-1-oxa-5,5,9,13-tetramethyl-7-(prop-2-yn-1-yl)-cyclohexadec-13-ene-2,6-dione;
(1S, 3S (E), 7S, 10R, 11S, 12S, 16R)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(4S, 7R, 8S, 9S, 13E/Z, 16R)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(1S, 3S, 7S, 10R, 11S, 12S, 16R)-7,11-dihydroxy-10-(2-oxacyclopropyl-1-methyl)-3-(2-methyl-benzothiazol-5-yl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-methyl-2-(2-methylthiazol-4-yl)ethenyl)-1-oxa-5,5,9,13-tetramethyl-7-(prop-2-en-1-yl)-cyclohexadec-13-ene-2,6-dione;
(1R, 3S (E), 7S, 10R, 11S, 12S, 16S)-7,11-dihydroxy-10-(prop-2-en-1-yl)-3-(1-methyl-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-methyl-2-(-(2-pyridyl)ethenyl)-1-oxa-5,5,9,13-tetramethyl-7-(prop-2-en-1-yl)-cyclohexadec-13-ene-2,6-dione;
(4S, 7R, 8S, 9S, 13E/Z, 16S (E))-4,8-dihydroxy-16-(1-fluoro-2-(2-methyloxazol-4-yl)ethenyl)-1-oxa-5,5,9,13-tetramethyl-7-(prop-2-yn-1-yl)-cyclohexadec-13-ene-2,6-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(3-methyl-but-2-en-1-yl)-cyclohexadec-13-ene-2,6-dione;
(1S, 3R, 7S, 10R, 11S, 12S, 16R)-7,11-dihydroxy-10-(prop-2-en-1-yl)-3-(2-methylbenzothiazol-5-yl)-8,8,12,16-tetramethyl-4-aza-17-oxa-bicyclo [14.1.0] heptadecane-5,9-dione;
(1R, 3S, 7S, 10R, 11S, 12S, 16S)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1R, 3S, 7S, 10R, 11S, 12S, 16S)-7,11-dihydroxy-10-(prop-2-yn-1-yl)-3-(2-methylbenzothiazol-5-yl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-en-1-yl)-3-(1-methyl-2-(2-pyridyl)ethenyl)-8,8,12,16-tetramethyl-4,17-dioxabicyclo [14.1.0] heptadecane-5,9-dione:
(1R, 3S (E), 7S, 10R, 11S, 12S, 16S)-7,11-dihydroxy-10-(but-3-en-1-yl)-3-(1-methyl-2-(2-pyridyl)ethenyl)-8,8,12,16-tetramethyl-4,17-dioxabicyclo [14.1.0] heptadecane-5,9-dione;
(4S, 7S, 8R, 9S, 13E/Z, 16S (E))-4,8-dihydroxy-16-(1-methyl-2-(2-pyridyl)ethenyl)-1-oxa-5,5,9,13-tetramethyl-7-(but-3-yn-1-yl)-cyclohexadec-13-ene-2,6-dione;
(1'S, 4S, 7R, 8S, 9S, 13E/Z, 16S (E))-4,8-dihydroxy-7-(prop-2-en-1-yl)-16-(1'-methyl-2'-(pyridin-2-yl)ethyl)-5,5,9,13-tetramethyl-1-oxa-hexadec-13-ene-2,6-dione;
(1'S, 4S, 7R, 8S, 9S, 13E/Z, 16S (E))-4,8-dihydroxy-7-(prop-2-en-1-yl)-16-(1'-methyl-2'-(pyridin-2-yl)ethyl)-5,5,9,13-tetramethyl-1-oxa-hexadec-13-ene-2,6-dione;
(1S/R, 3S (E), 7S, 10R, 11S, 12S, 16R/S)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1S/R, 3S (E), 7S, 10R, 11S, 12S, 16R/S)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(1R, 3S, 7S, 10R, 11S, 12S, 16S)-7,11-dihydroxy-10-(prop-2-en-1-yl)-3-(2-methylbenzoxazol-5-yl)-8,8,12,16tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-fluoro-2-(2-methyloxazol-4-yl)ethenyl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16R)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methylbenzothiazol-5-yl)-8,8,12,16-tetramethyl-4-aza-17-oxa-bicyclo [14.1.0] heptadecane-5,9-dione;
(1S, 3S, 7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione;
(4S, 7R, 8S, 9S, 13E/Z, 16S)-4,8-dihydroxy-16-(quinolin-7-yl)-1-oxa-5,5,9,13-tetramethyl-7-(prop-2-en-1-yl)-cyclohexadec-13-ene-2,6-dione;
(1S, 3S, 7S, 10R, 11S, 12S, 16R)-7,11-dihydroxy-10-(prop-2-en-1-yl)-3-(2-methylbenzothiazol-5-yl)-8,8,12,16-tetramethyl-4-aza-17-oxa-bicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzothiazol-5-yl)-8,8,12,16-tetramethyl-4-aza-17-oxa-bicyclo [14.1.0] heptadecane-5,9-dione;
(4S, 7R, 8S, 9S, 13E/Z, 16R)-4,8-dihydroxy-16-(2-methyl-benzoxazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16R)-4,8-dihydroxy-16-(2-methyl-benzoxazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16S)-4,8-dihydroxy-16-(2-methyl-benzoxazol-5-yl)-1-aza-5,5,9,13-tetramethyl-7-(prop-2-en-1-yl)-cyclohexadec-13-ene-2,6-dione;
(1R, 3R, 7S, 10R, 11S, 12S, 16S)-7,11-dihydroxy-10-(prop-2-en-1-yl)-3-(2-methylbenzoxazol-5-yl)-8,8,12,16tetramethyl-4-aza-17-oxa-bicyclo [14.1.0] heptadecane-5,9-dione;
(1S, 3R, 7S, 10R, 11S, 12S, 16R)-7,11-dihydroxy-10-(prop-2-en-1-yl)-3-(2-methylbenzoxazol-5-yl)-8,8,12,16tetramethyl-4-aza-17-oxa-bicyclo [14.1.0] heptadecane-5,9-dione;
(1R, 3R, 7S, 10R, 11S, 12S, 16S)-7,11-dihydroxy-10-(prop-2-en-1-yl)-3-(2-methylbenzoxazol-5-yl)-8,8,12,16tetramethyl-4-aza-17-oxa-bicyclo [14.1.0] heptadecane-5,9-dione;
(1S, 3S (E), 7S, 10R, 11S, 12S, 16R)-7,11-dihydroxy-10-(prop-2-en-1-yl)-3-(1-chloro-2-(2-methylthiazol-4-yl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-en-1-yl)-3-(1-chloro-2-(2-methylthiazol-4-yl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(2-oxacyclopropyl-1-methyl)-3-(1-chloro-2-(2-methyl-thiazol-4-yl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-en-1-yl)-3-(1-fluoro-2-(2-methyl-thiazol-4-yl)ethenyl)-16-hydroxymethyl-8,8,12-trimethyl-4,17-dioxabicyclo [14.1.0] heptadecane-5,9-dione;
(1S, 3S, 7S, 10R, 11S, 12S, 16R)-7,11-dihydroxy-10-(2-oxacyclopropyl-1-methyl)-3-(2-methyl-benzothiazol-5-yl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-7-(prop-2-en-1-yl)-16-(1-fluoro-2-(2-methyl-thiazol-4-yl)ethenyl)-13-hydroxymethyl-5,5,9-trimethyl-1-oxa-hexadec-13-ene-2,6-dione;
(1S, 3S (E), 7S, 10R, 11S, 12S, 16R)-7,11-dihydroxy-10-(prop-2-en-1-yl)-3-(1-fluoro-2-(2-methyl-thiazol-4-yl)ethenyl)-16-hydroxymethyl-8,8,12-trimethyl-4,17-dioxabicyclo [14.1.0] heptadecane-5,9-dione;
(4S, 7S, 8R, 9S, 13E/Z, 16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S, 3S, 7S, 10S, 11R, 12S, 16R)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1R, 3S, 7S, 10S, 11R, 12S, 16S)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(4R, 7S, 8R, 9R, 13E/Z, 16R)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1R, 3R, 7R, 10S, 11R, 12R, 16S)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1S, 3R, 7R, 10S, 11R, 12R, 16R)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-yn-1-yl)-3-(1-methyl-2-(2-pyridyl)ethenyl)-8,8,12,16-tetramethyl-4,17-dioxabicyclo[14.1.0] heptadecane-5,9-dione;
(1S, 3S (E), 7S, 10R, 11S, 12S, 16R)-7,11-dihydroxy-10-(but-3-en-1-yl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(prop-2-en-1-yl)-3-(1-methyl-2-(2-methylthiazol-4-yl)ethenyl)-8,8,12,16-tetramethyl-4,17-dioxabicyclo[14.1.0] heptadecane-5,9-dione;
(1S, 3S, 7S, 10R, 11S, 12S, 16R)-7,11-dihydroxy-10-(prop-2-en-1-yl)-3-(2-methylbenzoxazol-S-yl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(prop-2-en-1-yl)-3-(1-chloro-2-(2-methylthiazol-4-yl)ethenyl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-chloro-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(prop-2-yn-1-yl)-cyclohexadec-13-ene-2,6-dione;
(1S, 3S, 7S, 10R, 11S, 12S, 16R)-7,11-dihydroxy-10-(prop-2-yn-1-yl)-3-(2-methylbenzothiazol-5-yl)-8,8,12,16-tetramethyl-4,17-dioxabicyclo [14.1.0] heptadecane-5,9-dione;and/or
(4S, 7R, 8S, 9S, 13E/Z, 16S)-4,8-dihydroxy-16-(quinolin-7-yl)-1-oxa-5,5,9,13-tetramethyl-7-(prop-2-en-1-yl)-cyclohexadec-13-ene-2,6-dione.

In a very interesting embodiment, the Epothilone derivative is (1S, 3S, 7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

The Epothilone may be used in any amount in the compositions. It is of course desirable to apply high concentrations if possible, such as Epothilone in an amount of at least 0.05% by weight in the solid compositions, such as the lyophilisate. It is considered that a maximal content of Epothilone may be in the order of 2, 3, 4, 5 or 10% by weight dependent on the amount and type of reconstitution liquid. Typically, the amount of Epothilone is in a range between 0.05% and 10%, preferably 0.1% and 4% by weight, more preferably about 0.2 and 2% by weight in the lyophilisate. With respect to the reconstituted solution, the concentration ranges between 0.2 mg/ml and 10 mg/ml, preferably between 0.5 mg/ml and 5 mg/ml, such as about 1 mg/ml.

As mentioned, compositions of the invention comprise a cyclodextrin. It is envisaged that the effectiveness of combining a cyclodextrin as described herein with an Epothilone as described herein is consistent with the formation of a complex between the cyclodextrin and the Epothilone during manufacturing, or in the lyophilisate and/or in the reconstituted solution. More specifically, it is envisaged that the large size of the Epothilone of the invention compromises the formation of an inclusion complex wherein the Epothilone completely fits into the cavity of the cyclodextrin. It is more likely that the complex is only partially enclosed in the cavity of the cyclodextrin or not at all in contact with the cavity of the cyclodextrin. Therefore, the complex between a cyclodextrin according to the invention and an Epothilone according to the invention is preferably a partial inclusion complex or not an inclusion complex. For example in the form of a so-called sandwich complex. As mentioned, in some embodiments of the invention, the above- described complexes, such as partial inclusion complexes and sandwich complexes can be formed in either the lyophilisate and/or the reconstituted solution.

The term "cyclodextrin" is meant to define compounds comprising glucose units combined in a circular structure, namely compounds comprising 7 anhydro glucose units (β-cyclodextrin); 8 anhydro glucose units (γ-cyclodextrin) or 6 anhydro glucose units (α-cyclodextrin) as well as derivatives thereof. Each of the glucose units contains in 2-, 3-, and 6-position three hydroxy groups, which may be etherified or esterified, preferably etherified. Thus, the term " cyclodextrin derivative" encompasses herein etherified and esterified cyclodextrins. It is to be understood that the cyclodextrin may be completely or partially etherified or esterified, which means that all or only a part of the hydroxy groups are derivatised to form an ether or ester. Thus, at least 10%, such as least 20, 30, 40, 50, 60, 70, 80 or 90% of the alcohol groups may be alkylated or acylated. It should also be understood that no more than 40%, 30%, 20%, 10% or 5% of the 5% of the alcohol groups may be alkylated or acylated.

In preferred embodiments of the invention the cyclodextrin is β-cyclodextrin or a derivative thereof, such as an alkylated cyclodextrin, i.e. alkyl ether cyclodextrin. The alkyl may be of any carbon length, though preferably less than 10 carbon atoms, preferably less than 5 carbon atoms, such as an alkyl selected from methyl, ethyl, propyl, butyl or pentyl including branched chains thereof (iso-propyl).

In still preferred embodiments of the invention, the alkyl of the alkylated cyclodextrin contains a functional group such as hydroxy group and/or sulphur group. Thus, in preferred embodiments of the invention, the cyclodextrin derivative, such as a β-cyclodextrin derivative is etherified with hydroxyalkyl groups and/or sulfoalkyl groups resulting in hydroxyalkylated cyclodextrins (e. g.hydroxymethylated, hydroxyethylated, hydroxypropylated, hydroxybutylated, hydroxypentylated cyclodextrins), or sulfoalkylated cyclodextrins (e. g., sulfomethylated, sulfopenthylated, sulfopropylated, sulfobutylated, sulfopentylated cyclodextrins).

The etherification of a cyclodextrin with alkyl groups may be stated directly as degree of substitution (DS) per glucose unit, which as stated above is 3 for complete substitution. Partially etherified cyclodextrins are used within the invention, which comprise alkyl groups, such as hydroxyalkyl groups and sulfoalkyl groups as described above, up to a degree of substitution of 0.05 to 2.0, preferably 0.2 to 1.5. Most preferably the degree of substitution with alkyl groups is between about 0.5 and about 1.2 per glucose unit.

As mentioned, the combining of an Epothilone with a cyclodextrin is thought to result in a complex, such as a partial inclusion complex or a "sandwich" complex. In some embodiments, the molar ratio of Epothilone to cyclodextrin in a complex is preferably about 1:6 to 4:1, especially about 1:2 to 1:1. However, as a rule it is preferred to use the cyclodextrin in molar excess. Accordingly, the molar or weighed ratio between the cyclodextrin and the Epothilone may range between 10:1 and 1000:1, preferably between 20:1 and 800:1, more preferably between 50:1 and 500:1, such as between 75:1 and 400:1.

The cyclodextrin may be used in any amount in the compositions depending on the type of cyclodextrin. Preferably, the amount ranges between 10 and 99.8 % by weight in the solid compositions, such as in the lyophilisate. More preferably, the amount ranges between 30 and 98 %, most preferably between 50 and 98%, such as 69 and 96% by weight. With respect to the reconstituted solution, the concentration ranges between 10 mg/ml and 1000 mg/ml, preferably 50 mg/ml and 500 mg/ml, such as about 200 mg/ml.

In a preferred embodiment of the invention, the Epothilone is an Epothilone derivative according to formula I above and the cyclodextrin is a sulfoalkylated β-cyclodextrin, such as sulfomethylated, sulfopenthylated, sulfopropylated, sulfobutylated, sulfopentylated β-cyclodextrin, preferably sulfobutylated β-cyclodextrin. In other words, the cyclodextrin is sulfomethyl ether cyclodextrin, sulfoethyl ether cyclodextrin, sulfopropyl ether cyclodextrin, sulfobutyl ether cyclodextrin, sulfopentyl ether cyclodextrin, preferably sulfobutyl ether cyclodextrin.

In another preferred embodiment of the invention, the Epothilone is an Epothilone derivative according to formula I above and the cyclodextrin is a hydroxyalkylated β-cyclodextrin, such as hydroxymethylated, hydroxyethylated, hydroxypropylated, hydroxybutylated, hydroxypentylated β-cyclodextrin, preferably hydroxypropylated β-cyclodextrin, provided that said Epothilone and cyclodextrin is further combined with at least one pharmaceutically acceptable agent selected from a tonicifying agent, a filler and a buffering agent. In other words, the cyclodextrin may be hydroxymethyl ether cyclodextrin, hydroxyethyl ether cyclodextrin, hydroxypropyl ether cyclodextrin, hydroxybutyl ether cyclodextrin, hydroxypentyl ether cyclodextrin, preferably hydroxypropyl ether cyclodextrin.

As mentioned, the combination of a cyclodextrin as described herein and an Epothilone as described herein may form a complex, such as a partial inclusion complex or a "sandwich complex", either in solid composition, such as the lyophilisate or in re-constituted composition. Thus, a still further aspect of the invention relates to a complex between an Epothilone according to formula I above and a β-cyclodextrin, preferably a hydroxyalkylated β-cyclodextrin or a sulfoalkylated β-cyclodextrin mentioned above, the complex may be an inclusion complex wherein only a part of the Epothilone molecule is enclosed or the complex may be in the form of a "sandwich" complex.

Commercially available cyclodextrins of interest include alkyl and allyl derivatives, hydroxyalkyl derivatives such as gamma-cyclodextrin, hydroxypropyl-gamma-cyclodextrin, hydroxypropyl-beta-cyclodextrin, sulfobutylether-beta-cyclodextrin, methyl-beta-cyclodextrin, methylthioureido-beta-cyclodextrin, propanediamine-beta-cyclodextrin, ethanediamine-beta-cyclodextrin, hydroxyethylamino-beta-cyclodextrin.

As said compositions may further comprise at least one pharmaceutically acceptable agent selected from a tonicifying agent, a filler or a buffering agent.

The terms "tonicifying agent" and "tonicity modifier" are interchangeable terms referring to a compound that upon dissolving in the composition at the time of use, provides a tonicity within the physiological range of the blood, peritoneal fluid or other relevant body fluids. Obviously, the addition of a "tonicifying agent may also depend on whether the solvent for reconstitution comprises tonicity-modifying agents. The resulting solution, e.g. the reconstituted solution, may have an osmolality in the range of about 100 to 500 mOsm/kg H₂O. Preferably, the osmolality is in the range of about 150 to 900mOsm/kg H₂O, more preferably in the range of about 200 to 500 mOsm/kg H₂O, still more preferably in the range of about 250 to 350 mOsm/kg H₂O. Most suitable, the osmolality is in the range of about 280 to 320 mOsm/kg H₂O.

As used herein a tonicifying agent may be a polyol, such as mannitol, sorbitol and xylitol , preferably mannitol.

In some embodiments, the addition of a tonicity modifying agent is not required because the composition has the desired osmolality. The inventors have found that a suitable tonicity is achieved when used as the cyclodextrin, a sulfoalkyl ether β-cyclodextrin. Thus, in embodiments applying a sulfoalkyl ether β-cyclodextrin as the cyclodextrin, any tonicity modifying agent is not required or the sulfoalkyl ether β-cyclodextrin may be used in an amount not affecting the tonicity of the composition.

In a preferred embodiment of the invention, at least one further pharmaceutically acceptable excipient, which need to be to be added to the combination of an Epothilone and a cyclodextrin is mannitol, sorbitol and/or xylitol. The addition of mannitol, sorbitol and/or xylitol may serve different functions. In general it is known that mannitol is a filler (bulking agent) suitable for lyophilised compositions, and as mentioned mannitol has also tonicity modifying activity when dissolved in a liquid composition, such as the composition resulting from reconstitution of the lyophilisate with a suitable solvent.

The term "cryoprotectants" as used herein generally include agents, which provide stability to the Epothilone from freezing-induced stresses. Examples of cryoprotectants include cyclodextrins such as β-cyclodextrins and derivatives thereof, and include polyols such as, for example, mannitol, as well as pH regulators. The pH regulators include amines, such as trometamol, mineral acids, organic acids, such as hydrochloric acid. Additionally surfactants can be used as cryoprotectants. Examples of surfactants include amines such as, trometamol. Cryoprotectants also contribute to the tonicity of the formulations. Cryoprotectants may also have lyoprotectant effects.

The term "lyoprotectant" as used herein includes agents that provide stability to the Epothilone during water removal in the drying process, such as during lyophilisation process. Examples of lyoprotectants include saccharides, in particular sugar alcohols in particular mannitol. Saccharides of interest are di- and tri-saccharides such as sucrose, dextrose, lactose, maltose and/or trehalose.

The term "filler" or "bulking agent" is interchangeable terms denoted to mean an agent providing good lyophilised cake properties, which form a pharmaceutically elegant product, which help the Epothilone or a derivative thereof to overcome various stresses, shear/freezing for example, associated with lyophilization processes. Furthermore, a filler helps to maintain the therapeutically activity levels during the freeze-drying process and subsequent storage. Typical examples on bulking agents include cyclodextrins, sugar alcohols, such as mannitol. These agents may also contribute to the tonicity of the formulations.

It is envisaged that the required amount of tonicity modifier, bulking agent, lyoprotectant, or cryoprotectant may depend on several factors such as the desired osmolality, stability, lyophilisation process and reconstitution characteristics. With respect to mannitol, it has been found that the required amount ranges between 0.5 and 50 % by weight in the solid compositions, such as in the lyophilisate. More preferably, the amount ranges between 2 and 20 %. With respect to the reconstituted solution, the concentration ranges between 1 mg/ml and 200 mg/ml, preferably between 2 mg/ml and 100 mg/ml, more preferably between 5 mg/ml and 50 mg/ml, such as about 20 mg/ml.

As said the compositions of the invention may further comprise a pH regulator, such as a buffering compound. It is envisaged that a pH regulator may be applied in order to further stabilise the Epothilones that are easily hydrolysed in aqueous solution having pH above neutral.

The term "pH regulator" is meant to encompass compounds that are suitable for keeping/maintaining the pH in the range of 4 to 9 in the reconstituted solution. As used herein, the pH regulator preferably maintain the pH in the range of 5 to 8, more preferably in the range of 6 to 7.5. Therefore, in a still further interesting embodiment of the invention, the pH of the compositions is kept within the pH range of within of 5 to 8, more preferably in the range of 6 to 7.5. That is to say that the pH in the Epothilone solution at the time before removing the moisture content, e.g. before freeze-drying, should be kept within a pH of 5 to 8. Advantageously, this pH range is also within the desired physiological range, thereby causing no harm to the user upon administering the composition by parenteral means. Preferably the pH is about neutral, such as close to a pH of 7.4.

Typical examples of pH regulators are TRIS, the acid form or salts of citric acid, acetic acid, histidine, malic acid, phosphoric acid, tartaric acid, succinic acid, MES, HEPES, imidazole, lactic acid, glutaric acid and glycylglycine. In one embodiment TRIS is used alone and in another embodiment TRIS is used together with hydrochloric acid.

By the term "TRIS" is understood 2-Amino-2-hydroxymethyl-1,3-propandiol, which also are known under the names trometamol; trimethylol aminomethane; tris(hydroxymethyl)aminomethane; trismanine; tris buffer; tromethane; THAM; Talastrol; Tris Amino and Tromethamine.

The pH-regulator may be used in any amount. Though, it has been found that the required amount ranges between 0.05 and 4.2 % in the solid compositions, such as in the lyophilisate. More preferably, the amount ranges between 0.2 and 1.5% by weight. With respect to the reconstituted solution, the concentration ranges between 0.1 mg/ml and 10 mg/ml, preferably between 0.2 mg/ml and 5 mg/ml, more preferably between 0.5 mg/ml and 3 mg/ml, such as about 1.2 mg/ml. Furthermore, the pH regulator may be used together with an acid or base, such as hydrochloric acid. The acid may be used in any amount. Though, it has been found that the required amount ranges between 0.01 and 0.9 % in the solid compositions, such as in the lyophilisate. More preferably, the amount ranges between 0.05 and 0.3 %. With respect to the reconstituted solution, the concentration ranges between 0.03 mg/ml and 2 mg/ml, preferably between 0.05 mg/ml and 1 mg/ml, more preferably between 0.1 mg/ml and 0.6 mg/ml, such as about 0.3 mg/ml.

It should be understood that the compositions of the invention are made without adding a surfactant. Thus, in interesting embodiments of the invention, the composition excludes a surfactant or at least is substantially free of surfactant. The term "surfactant" generally includes an agent, which protect the Epothilone or a derivative thereof from air/solution interface-induced stresses and solution/surface induced-stresses. For example, a surfactant may protect the Epothilone or a derivative thereof from aggregation. Suitable surfactants may include certain amines, polysorbate or poloxamer such as Tween 20, Tween 80, or poloxamer 188.

It should be understood that the embodiments described herein may be combined in any suitable manner. Preferred embodiment of the invention includes a composition comprising an Epothilone as defined herein, a cyclodextrin as defined herein and at least one pharmaceutically acceptable excipient selected from the group consisting of mannitol; sorbitol; xylitol; 2-Amino-2-hydroxymethyl-1,3-propandiol; the acid form or salts of citric acid, acetic acid, histidine, malic acid, phosphoric acid, tartaric acid, succinic acid, MES, HEPES, imidazole, lactic acid, glutaric acid and glycylglycine, preferably mannitol and/or TRIS.

Still preferably embodiments of the inventions are compositions comprising;
i) Epothilone derivative in an amount of 0.1 - 2% by weight, preferably 0.2 - 1% by weight,
ii) hydroxyalkyl- β-cyclodextrin, preferably 2-Hydroxypropyl-β-cyclodextrin in an amount of 50 - 99% by weight, preferably 70 - 95% by weight,
iii) mannitol, xylitol or sorbitol, preferably mannitol in an amount of 0 - 50% by weight, preferably 1 - 20% by weight, more preferably 2-15% by weight,
iv) pH regulator, preferably Trometamol in an amount of 0 - 2% by weight, preferably 0.1 - 1% by weight,
v) Hydrochloric acid in an amount of 0 - 1% by weight, preferably 0.05 - 0.5% by weight.

Still preferably embodiments of the inventions are compositions comprising;
i) Epothilone derivative in an amount of 0.01 - 2% by weight, preferably 0.02 - 1% by weight,
ii) sulfoalkyl- β-cyclodextrin, preferably sulfobutyl-β-cyclodextrin in an amount of 50 - 99.9% by weight, preferably 85 - 99.5% by weight,
iii) pH regulator, preferably Trometamol in an amount of 0 - 2% by weight, preferably 0.1 - 1% by weight, and
iv) Hydrochloric acid in an amount of 0 - 1% by weight, preferably 0.05 - 0.5% by weight.

Provided by the present invention are compositions with good chemical stability with respect to the Epothilone despite the fact that Epothilones are easily hydrolysed. The term "good chemical stability" is meant to describe that the hydrolysis or otherwise chemical degradation of the Epothilone is minimised during storage or production of the compositions so that substantial preservation of the Epothilone is maintained.

Like-wise the chemical stability of the solid composition, such as the lyophilisate, is high. The stability is typically determined by storing the lyophilisate in a glass vial stopped with rubber stoppers and the amount of degradation product formed over a period of up to 1, 3, 6, or 9 months is analyzed by determining the formation of degradation products in each of the reconstituted solutions as a function of time and temperature. The concentration of Epothilone and its degradation products is determined using quantitative assays, such as by HPLC.

For example the stability of lyophilisate is such that less than 15% of the initial amount of Epothilone in the composition is degraded over a period up to 3 months when the composition is stored as sealed in the dark at 2°C to 8°C. Preferably less than 10%, such as less than 5% of the initial amount of Epothilone in the composition is degraded at the stated conditions.

The term "initial content" relates to the amount of Epothilone added to a composition at the time of preparation. The concentration given herein (mg/ml) refers to either the concentration in the solution of Epothilone before removing the moisture (e.g. before freeze-drying) or is referred as % w/w, which then relates to the concentration in the solid composition, e.g. the lyophilised cake.

A still further aspect of the invention relates to the manufacturing of a solid composition, such as a lyophilisate, according to the invention, which may be formed from solutions (hereinafter referred to as "original solutions") comprising an Epothilone described herein, a cyclodextrin described herein and optionally at least one further pharmaceutically acceptable excipient as defined herein above.

The method for producing a composition of the invention comprises the steps of
a) dissolving an Epothilone as defined herein in an organic solvent, such as an alcohol (preferably ethanol); and
b) dissolving a cyclodextrin as defined herein in aqueous solution, optionally together with at least one further pharmaceutical acceptable ingredient as defined herein, such as mannitol and /or tromethamol; optionally
c) adjusting the pH of the resulting mixture of b) to a pH ranging between 5 and 9, preferably 6 and 8, such as about 7.4 using an inorganic acid, such as hydrochloric acid; and
d) mixing the resulting solvents a) and b) or a) and c); and optionally
e) carrying out sterile filtering of d) to achieve the so-called "original solution"
f) drying the solution so as to remove the solvent resulting in a solid composition.

Suitably, the drying process is provided by lyophilisation or rotation evaporator.

The compositions of the invention may be used for the treatment of a disease or condition associated with cell growth, division and/or proliferation, such as for treating malignant tumors in an individual in need thereof. As applications, there can be mentioned, for example, the therapy of ovarian, stomach, colon, adeno-, breast, lung, head and neck carcinomas, malignant melanoma, acute lymphocytic, myelocytic leukaemia, bonemetastasis, and brain tumours. The compositions according to the invention are also suitable for treatment of chronic inflammatory diseases, such as, for example, psoriasis or arthritis. It follows that the compositions may be used for the preparation of a medicament for the treatment of the above-mentioned diseases.

Another further aspect of the invention relates to methods of treating diseases and conditions associated with cell growth, division and/or proliferation in patients comprising administering to the patient a therapeutically effective of one or more compound of formula I using the compositions of the present invention, wherein said compositions are administered by intravenous infusion over a period of about 30 minutes in a dose ranging from 10 mg/m² to 35 mg/m², preferably from 16 mg/m² to 29 mg/m², most preferably 22 mg/m². The methods of the present invention also encompass dosing schedules, such as administration of the compositions of the invention to the patient once every 3 weeks or weekly for 3 weeks followed by one week of recovery or rest. The compositions are administered until progression or until the occurrence of unacceptable toxicities (i.e. Dose-Limiting Toxicities). A further aspect of the invention provides compositions that upon parenteral administration, such as by intravenous injection, result in a high Maximum Tolerated Dose (MTD), such as above 10 mg/m², preferably above 16 mg/m², more preferably above 22 mg/m². The MTD of an Epothilone administered in the form of a reconstituted composition of the present invention, may be observed in standard animal tests and in clinical trials.

### EXAMPLES

### Example 1

Composition comprising hydroxypropyl-β-cyclodextrin and the Epothilone derivative*: 1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

| **Ingredients** | **Lyophilisate (mg)** | **Lyophilisate (%)** | **Reconstituted composition (mg/ml)** |
|---|---|---|---|
| Epothilone derivative* | 10.500 | 0.449 | 1.000 |
| 2-Hydroxypropyl-β-cyclodextrin | 2100.000 | 89.879 | 200.000 |
| Mannitol | 210.000 | 8.988 | 20.000 |
| Trometamol | 12.705 | 0.544 | 1.210 |
| Hydrochloric acid | 3.267 | 0.140 | 0.311 |
| Total | 2336.472 | | |

The freeze-dried product (lyophilisate) is reconstituted by adding 8.8 ml of water for injection.

### Example 2

Composition comprising sulfobutyl-ether-β-cyclodextrin and the Epothilone derivative*: 1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

| **Ingredients** | **Quantity in the lyophilised mass mg** | **Concentration after reconstitution mg/ml** |
|---|---|---|
| Epothilone * | 5.500 | 1.000 |
| Sulfobutyl-ether-β-cyclodextrin | 1,100.000 | 2000.000 |
| Trometamol | 6.655 | 1.210 |
| Hydrochloric acid | ad pH 7.4 (in solution) | ad pH 7.4 |

### Example 3

*Manufacturing process for an "original solution" from where a lyophilisate is prepared*

| **Ingredients** | **Quantity in g** |
|---|---|
| Epothilone* | 14.250 |
| Hydroxypropyl-β-cyclodextrin | 2850.000 |
| Mannitol | 285.000 |
| Trometamol | 17.243 |
| Hydrochloric acid | 44.336 |
| Ethanol 96% - processing aid | 173.850 |
| Water for injection - processing aid | 11862.822 |

| | |
|---|---|
| *(1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione. | |

In the first production step the Epothilone* is dissolved in ethanol 96%. In the second production step hydroxypropyl-β-cyclodextrin is dissolved in with water for injection. Trometamol is subsequently added to the cyclodextrin solution and then mannitol.
The resulting solution of hydroxypropyl-β-cyclodextrin, trometamol and mannitol is adjusted to pH 7.4 by adding diluted hydrochloric acid.

Then the Epothilone solution and the cyclodextrin solution are combined and freeze dried in order to obtain a solid composition as shown in Example 1.

### Example 4

Stability data of Epothilone* (1S, 3S, 7S, 10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione) in aqueous solutions containing hydroxypropyl-beta-cyclodextrin.

Freeze-dried preparations as shown in Example 1 were reconstituted with water for injection and the pH was then adjusted to pH values between 6.1 and 13.6 using HCl or NaOH. The solutions were left at room temperature or 40°C and assay and purity were measured at intervals using HPLC.

The degradation rate constant (K) of the Epothilone* decreases as the pH value approaches the neutral pH. The degradation rate is at minimum at pH 7.

| **pH** | **K at room temperature** | **K (40°)** |
|---|---|---|
| 9 | 4.5 10⁻³ h⁻¹ | |
| 8.0 | 1.1 10⁻³ h⁻¹ | 4.9 10⁻³ h⁻¹ |
| 7.5 | 0.7 10⁻³ h⁻¹ | |
| 6.9 | 0.1 10⁻³ h⁻¹ | 1.2 10⁻³ h⁻¹ |
| 6.3 | 0.5 10⁻³ h⁻¹ | 1.7 10⁻³ h⁻¹ |
| 6.1 | 0.8 10⁻³ h⁻¹ | |

### Example 5

Determination of the apparent equilibrium stability constant of the complex between Epothilone*(1S, 3S, 7S, 10R, 11S, 12S, 16R)-7,11-dihydroxy-3-(2-methylbenzothiazol-5-yl)-10-(prop-2-en-1-yl)-8,8,12,16-tetramethyl-4,17-dioxabicyclo[14,1,0]heptadecane-5,9-dione) and hydroxypropyl-beta-cyclodextrin on aqueous solution.

The phase solubility diagram technique (PSD) was applied at 25°C and the stability constant of an assumed 1:1 complex are K'= 484.5M⁻¹. The solubility of the Epothilone* was Sₒ= 4.3 10⁻⁵ mol/l (0.23g/l).

### Example 6

Stability of the lyophilisate and of reconstituted lyophilisate containing Epothilone* at various storage conditions:

### Stability of the Lyophilisate

See Figures 1 and 2

### Stability of the Reconstituted Lyophilisate

| | **Content Epothilone* [mg/mL]** | **pH** | **Osmolality [mmol/kg]** | **Density [g/mL]** | **Color** |
|---|---|---|---|---|---|
| Start filtered | 0.99 | 7.41 | 355 | 1.0745 | ≥ Y7 |
| | 1.02 | | | | |
| 6 h | 1.01 | 7.41 | 350 | 1.0746 | ≥Y7 |
| filtered | 1.01 | | | | |
| 24 h | 1.01 | 7.42 | 351 | 1.0750 | ≥ Y7 |
| filtered | 1.00 | | | | |

### Example 7

### Administration of the Epothilone* Using the Compositions of the Invention

Patients and Methods: Patients with histologically confirmed advanced solid tumors that were resistant or refractory to conventional antineoplastic treatment were eligible for the trial. They received treatment with Epothilone* as 30 minutes intravenous infusion in 3 weeks intervals. Treatment was continued until progression or the occurrence of unacceptable toxicities. The starting dose was 0,6 mg/m². Doses were escalated using a modified Fibonacci design.

Results: 47 patients have been enrolled at 12 different dose levels up to 29 mg/m². Dose limiting toxicities observed were CTC Grade 3 peripheral neuropathy at 16 mg/m² and CTC Grade 4 ataxia at 29 mg/m². No other DLTs were observed. Hematologic toxicities of maximum CTC Grade 2 were infrequent. Most common side effect was peripheral sensory neuropathy, mostly of Grade 1-2. No Grade 3-4 non-hematological toxicities were reported, with the exception of the previously-mentioned DLTs. Results show anti-tumor activity (including objective responses) in patients with breast cancer, NSCLC, cholangiocarcinoma, uveal melanoma and head and neck cancer and that the Epothilone* can be administered every 3 weeks at doses up to 29 mg/m² without severe toxicity.

## Claims

1. A composition comprising an Epothilone, a cyclodextrin and at least one pharmaceutically acceptable excipient selected from the group consisting of mannitol; sorbitol; xylitol; 2-Amino-2-hydroxymethyl-1,3-propandiol; the acid form or salts of citric acid, acetic acid, histidine, malic acid, phosphoric acid, tartaric acid, succinic acid, MES, HEPES, imidazole, lactic acid, glutaric acid and glycylglycine.

2. A composition comprising an Epothilone derivative of formula I and a cyclodextrin, wherein formula I is : 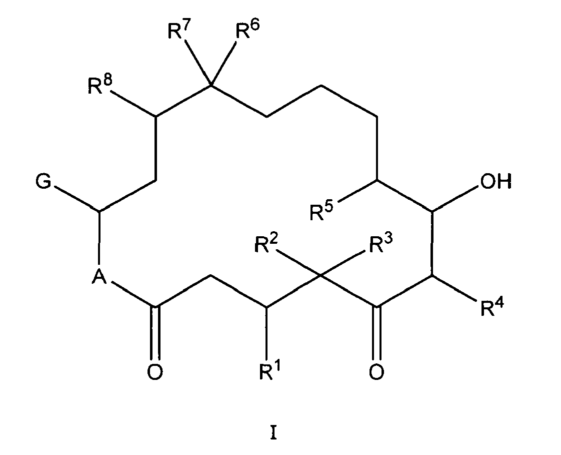 wherein
R¹ means hydrogen, OR^{1a}, or Halogen, where R^{1a} is hydrogen, SO₂-alkyl, SO₂₋aryl, or SO₂-aralkyl,
R², R³ are independently C₁-C₁₀ alkyl,
R⁴ means -(CH₂)ᵣ-C≡C-(CH₂)ₚ-R^{4a}, -(CH₂)ᵣ-CH=CH-(CH₂)ₚ-R^{4a}, 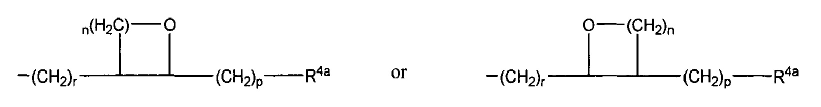
n means 0 to 5,
r is 0 to 4,
p is 0 to 3,
R^{4a} means hydrogen, C₁-C₁₀ alkyl, C₆-C₁₂ aryl or C₇-C₂₀ aralkyl;C₁-C₁₀ acyl, or, if p > 0, additionally a group OR^{4b}, R^{4b} means hydrogen or a protective group PG;
R⁵ means C₁-C₁₀ alkyl,
R⁶ means hydrogen or optionally substituted C₁-C₁₀ alkyl,
R⁷, R⁸ each mean a hydrogen atom, or taken together an additional bond or taken together an oxygen atom,
G means a group X=CR⁹- or a bi- or tricyclic aryl radical,
R⁹ means hydrogen, halogen, CN, or a C₁-C₂₀ alkyl,
X means a grouping CR¹⁰R¹¹,
whereby
R¹⁰, R¹¹ are the same or different and stand for hydrogen, a C₁-C₂₀ alkyl, C₆-C₁₂ aryl, or C₇₋₂₀ aralkyl radical each optionally substituted; or R¹⁰ and R¹¹ together with the methylene carbon atom jointly stand for a 5- to 7-membered carbocyclic ring;
A means a group -O- or -NR¹²-,
R¹² means hydrogen or C₁-C₁₀ alkyl.

3. The composition according to claim 1, wherein the Epothilone is selected from Epothilone A, Epothilone B, Epothilone C, Epothilone D, and a derivative thereof.

4. The composition according to claim 3, wherein the Epothilone is an Epothilone B derivative.

5. The composition according to claim 3, wherein the Epothilone is a Epothilone derivative of formula I as defined in claim 2 and wherein R¹,R², R³,R⁴,n, r, p, R^{4a}, R^{4b}, R⁵, R⁶, R⁷, R⁸, G, R⁹, X, R¹⁰, R¹¹, A, R¹² are as defined in claim 2.

6. The composition according to any one of preceding claims, wherein the Epothilone is an Epothilone derivative selected from the group consisting of:
(1S, 3S (E), 7S, 10R, 11S, 12S, 16R)-7,11-dihydroxy-10-(prop-2-yn-1-yl)-3-(1-methyl-2-(2-methylthiazol-4-yl)ethenyl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzoxazol-5-yl)-8,8,12,16-tetramethyl-4,17-dioxabicyclo [14.1.0] heptadecane-5,9-dione;
(1S, 3S (E), 7S, 10R, 11R, 12S, 16R)-7,11-dihydroxy-10-(prop-2-yn-1-yl)-3-(1-fluoro-2-(2-methyloxazol-4-yl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-chloro-2-(2-methylthiazol-4-yl)ethenyl)-1-oxa-5,5,9,13-tetramethyl-7-(prop-2-en-1-yl)-cyclohexadec-13-ene-2,6-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(4S, 7R, 8S, 9S, 13E/Z, 16S (E))-4,8-dihydroxy-16-(1-methyl-2-(2-pyridyl)ethenyl)-1-oxa-5,5,9,13-tetramethyl-7-(but-3-yn-1-yl)-cyclohexadec-13-ene-2,6-dione;
(4S, 7R, 8S, 9S, 13E/Z, 16S (E))-4,8-dihydroxy-16-(1-methyl-2-(2-pyridyl)ethenyl)-1-oxa-5,5,9,13-tetramethyl-7-(but-3-en-1-yl)-cyclohexadec-13-ene-2,6-dione;
(1S, 3S (E), 7S, 10R, 11S, 12S, 16R)-7,11-dihydroxy-10-(prop-2-en-1-yl)-3-(1-methyl-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-methyl-2-(2-pyridyl)ethenyl)-1-oxa-5,5,9,13-tetramethyl-7-(prop-2-yn-1-yl)-cyclohexadec-13-ene-2,6-dione;
(4S, 7R, 8S, 9S, 13E/Z, 16S (E))-4,8-dihydroxy-16-(1-fluoro-2-(2-methylthiazol-4-yl)ethenyl)-1-oxa-5,5,9,13-tetramethyl-7-(prop-2-yn-1-yl)-cyclohexadec-13-ene-2,6-dione;
(1S, 3S (E), 7S, 10R, 11S, 12S, 16R)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(4S, 7R, 8S, 9S, 13E/Z, 16R)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(1S, 3S, 7S, 10R, 11S, 12S, 16R)-7,11-dihydroxy-10-(2-oxacyclopropyl-1-methyl)-3-(2-methyl-benzothiazol-5-yl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-methyl-2-(2-methylthiazol-4-yl)ethenyl)-1-oxa-5,5,9,13-tetramethyl-7-(prop-2-en-1-yl)-cyclohexadec-13-ene-2,6-dione;
(1R, 3S (E), 7S, 10R, 11S, 12S, 16S)-7,11-dihydroxy-10-(prop-2-en-1-yl)-3-(1-methyl-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-16-(1-methyl-2-(-(2-pyridyl)ethenyl)-1-oxa-5,5,9,13-tetramethyl-7-(prop-2-en-1-yl)-cyclohexadec-13-ene-2,6-dione;
(4S, 7R, 8S, 9S, 13E/Z, 16S (E))-4,8-dihydroxy-16-(1-fluoro-2-(2-methyloxazol-4-yl)ethenyl)-1-oxa-5,5,9,13-tetramethyl-7-(prop-2-yn-1-yl)-cyclohexadec-13-ene-2,6-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(3-methyl-but-2-en-1-yl)-cyclohexadec-13-ene-2,6-dione;
(1S, 3R, 7S, 10R, 11S, 12S, 16R)-7,11-dihydroxy-10-(prop-2-en-1-yl)-3-(2-methylbenzothiazol-5-yl)-8,8,12,16-tetramethyl-4-aza-17-oxa-bicyclo [14.1.0] heptadecane-5,9-dione;
(1R, 3S, 7S, 10R, 11S, 12S, 16S)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1R, 3S, 7S, 10R, 11S, 12S, 16S)-7,11-dihydroxy-10-(prop-2-yn-1-yl)-3-(2-methylbenzothiazol-5-yl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-en-1-yl)-3-(1-methyl-2-(2-pyridyl)ethenyl)-8,8,12,16-tetramethyl-4,17-dioxabicyclo [14.1.0] heptadecane-5,9-dione:
(1R, 3S (E), 7S, 10R, 11S, 12S, 16S)-7,11-dihydroxy-10-(but-3-en-1-yl)-3-(1-methyl-2-(2-pyridyl)ethenyl)-8,8,12,16-tetramethyl-4,17-dioxabicyclo [14.1.0] heptadecane-5,9-dione;
(4S, 7S, 8R, 9S, 13E/Z, 16S (E))-4,8-dihydroxy-16-(1-methyl-2-(2-pyridyl)ethenyl)-I-oxa-5,5,9,13-tetramethyl-7-(but-3-yn-1-yl)-cyclohexadec-13-ene-2,6-dione;
(1'S, 4S, 7R, 8S, 9S, 13E/Z, 16S (E))-4,8-dihydroxy-7-(prop-2-en-1-yl)-16-(1'-methyl-2'-(pyridin-2-yl)ethyl)-5,5,9,13-tetramethyl-1-oxa-hexadec-13-ene-2,6-dione;
(1'S, 4S, 7R, 8S, 9S, 13E/Z, 16S (E))-4,8-dihydroxy-7-(prop-2-en-1-yl)-16-(1'-methyl-2'-(pyridin-2-yl)ethyl)-5,5,9,13-tetramethyl-1-oxa-hexadec-13-ene-2,6-dione;
(1S/R, 3S (E), 7S, 10R, 11S, 12S, 16R/S)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1S/R, 3S (E), 7S, 10R, 11S, 12S, 16R/S)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(2-oxacyclopropyl-1-methyl)-3-(1-methyl-2-(2-pyridyl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-fluoro-2-(2-methylthiazol-4-yl)ethenyl)-8,8,12,16-tetramethyl-4,17-dioxabicyclo [14.1.0] heptadecane-5,9-dione;
(1R, 3S, 7S, 10R, 11S, 12S, 16S)-7,11-dihydroxy-10-(prop-2-en-1-yl)-3-(2-methylbenzoxazol-5-yl)-8,8,12,16tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-fluoro-2-(2-methyloxazol-4-yl)ethenyl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16R)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;
(1R, 3S, 7S, 10R, 11S, 12S, 16S)-7,11-dihydroxy-10-(prop-2-en-1-yl)-3-(2-methylbenzothiazol-5-yl)-8,8,12,16-tetramethyl-4-aza-17-oxa-bicyclo [14.1.0] heptadecane-5,9-dione;
(1S, 3S, 7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione;
(4S, 7R, 8S, 9S, 13E/Z, 16S)-4,8-dihydroxy-16-(quinolin-7-yl)-1-oxa-5,5,9,13-tetramethyl-7-(prop-2-en-1-yl)-cyclohexadec-13-ene-2,6-dione;
(1S, 3S, 7S, 10R, 11S, 12S, 16R)-7,11-dihydroxy-10-(prop-2-en-1-yl)-3-(2-methylbenzothiazol-5-yl)-8,8,12,16-tetramethyl-4-aza-17-oxa-bicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzothiazol-5-yl)-8,8,12,16-tetramethyl-4-aza-17-oxa-bicyclo [14.1.0] heptadecane-5,9-dione;
(4S, 7R, 8S, 9S, 13E/Z, 16R)-4,8-dihydroxy-16-(2-methyl-benzoxazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16R)-4,8-dihydroxy-16-(2-methyl-benzoxazol-5-yl)-1-aza-5,5,9,13-tetramethyl-7-(prop-2-en-1-yl)-cyclohexadec-13-ene-2,6-dione;
(4S, 7R, 8S, 9S, 13E/Z, 16S)-4,8-dihydroxy-16-(2-methyl-benzoxazol-5-yl)-1-aza-5,5,9,13-tetramethyl-7-(prop-2-en-1-yl)-cyclohexadec-13-ene-2,6-dione;
(1R, 3R, 7S, 10R, 11S, 12S, 16S)-7,11-dihydroxy-10-(prop-2-en-1-yl)-3-(2-methylbenzoxazol-5-yl)-8,8,12,16tetramethyl-4-aza-17-oxa-bicyclo [14.1.0] heptadecane-5,9-dione;
(1S, 3R, 7S, 10R, 11S, 12S, 16R)-7,11-dihydroxy-10-(prop-2-en-1-yl)-3-(2-methylbenzoxazol-5-yl)-8,8,12,16tetramethyl-4-aza-17-oxa-bicyclo [14.1.0] heptadecane-5,9-dione;
(1R, 3R, 7S, 10R, 11S, 12S, 16S)-7,11-dihydroxy-10-(prop-2-en-1-yl)-3-(2-methylbenzoxazol-5-yl)-8,8,12,16tetramethyl-4-aza-17-oxa-bicyclo [14.1.0] heptadecane-5,9-dione;
(1S, 3S (E), 7S, 10R, 11S, 12S, 16R)-7,11-dihydroxy-10-(prop-2-en-1-yl)-3-(1-chloro-2-(2-methylthiazol-4-yl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-en-1-yl)-3-(1-chloro-2-(2-methylthiazol-4-yl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(2-oxacyclopropyl-1-methyl)-3-(1-chloro-2-(2-methyl-thiazol-4-yl)ethenyl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-en-1-yl)-3-(1-fluoro-2-(2-methyl-thiazol-4-yl)ethenyl)-16-hydroxymethyl-8,8,12-trimethyl-4,17-dioxabicyclo [14.1.0] heptadecane-5,9-dione;
(1S, 3S, 7S, 10R, 11S, 12S, 16R)-7,11-dihydroxy-10-(2-oxacyclopropyl-1-methyl)-3-(2-methyl-benzothiazol-5-yl)-8,8,12,16-tetramethyl-4,17-dioxabicyclo [14.1.0] heptadecane-5,9-dione;
(4S, 7R, 8S, 9S, 13E/Z, 16S (E))-4,8-dihydroxy-7-(prop-2-en-1-yl)-16-(1-fluoro-2-(2-methyl-thiazol-4-yl)ethenyl)-13-hydroxymethyl-5,5,9-trimethyl-1-oxa-hexadec-13-ene-2,6-dione;
(1S, 3S (E), 7S, 10R, 11S, 12S, 16R)-7,11-dihydroxy-10-(prop-2-en-1-yl)-3-(1-fluoro-2-(2-methyl-thiazol-4-yl)ethenyl)-16-hydroxymethyl-8,8,12-trimethyl-4,17-dioxabicyclo [14.1.0] heptadecane-5,9-dione;
(4S, 7S, 8R, 9S, 13E/Z, 16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S, 3S, 7S, 10S, 11R, 12S, 16R)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1R, 3S, 7S, 10S, 11R, 12S, 16S)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(4R, 7S, 8R, 9R, 13E/Z, 16R)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1R, 3R, 7R, 10S, 11R, 12R, 16S)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1S, 3R, 7R, 10S, 11R, 12R, 16R)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-yn-1-yl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(but-3-en-1-yl)-3-(1-methyl-2-(2-pyridyl)ethenyl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(prop-2-en-1-yl)-3-(1-methyl-2-(2-methylthiazol-4-yl)ethenyl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzoxazol-5-yl)-8,8,12,16-tetramethyl-4,17-dioxabicyclo [14.1.0] heptadecane-5,9-dione;
(1S, 3S (E), 7S, 10R, 11S, 12S, 16R)-7,11-dihydroxy-10-(prop-2-en-1-yl)-3-(1-chloro-2-(2-methylthiazol-4-yl)ethenyl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(2-methylbenzothiazol-5-yl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1S, 3S, 7S, 10R, 11S, 12S, 16R)-7,11-dihydroxy-10-(prop-2-en-1-yl)-3-(quinolin-7-yl)-8,8,12,16-tetramethyl-4,17-dioxabicyclo [14.1.0] heptadecane-5,9-dione;
(1R, 3S (E), 7S, 10R, 11S, 12S, 16S)-7,11-dihydroxy-10-(prop-2-yn-1-yl)-3-(1-chloro-2-(2-methylthiazol-4-yl)ethenyl)-8,8,12,16-tetramethyl-4,17-dioxabicyclo [14.1.0] heptadecane-5,9-dione;
(4S, 7R, 8S, 9S, 13E/Z, 16S)-4,8-dihydroxy-16-(2-methyl-benzothiazol-5-yl)-1-oxa-5,5,9,13-tetramethyl-7-(prop-2-yn-1-yl)-cyclohexadec-13-ene-2,6-dione;
(1S, 3S, 7S, 10R, 11S, 12S, 16R)-7,11-dihydroxy-10-(prop-2-yn-1-yl)-3-(2-methylbenzothiazol-5-yl)-8,8,12,16-tetramethyl-4,17-dioxabicyclo [14.1.0] heptadecane-5,9-dione;and/or
(4S, 7R, 8S, 9S, 13E/Z, 16S)-4,8-dihydroxy-16-(quinolin-7-yl)-1-oxa-5,5,9,13-tetramethyl-7-(prop-2-en-1-yl)-cyclohexadec-13-ene-2,6-dione.

7. The composition according to any one of preceding claims, wherein the Epothilone is (1S, 3S, 7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione.

8. The composition according to any one of preceding claims, wherein the cyclodextrin is selected from the group consisting of α-cyclodextrin, β-cyclodextrin, γ-cyclodextrin and derivatives thereof.

9. The composition according to claim 8, wherein the cyclodextrin is β-cyclodextrin or a derivative thereof.

10. The composition according to claim 9, wherein the cyclodextrin is an alkyl ether β-cyclodextrin.

11. The composition according to claim 10, wherein the cyclodextrin a hydroxyalkylated -β-cyclodextrin.

12. The composition according to claim 11, wherein the cyclodextrin is 2-hydroxypropyl-β-cyclodextrin.

13. The composition according to any one of claims 1 to 10, wherein the cyclodextrin is a sulfoalkylated cyclodextrin.

14. The composition according to claim 13, wherein the sulfoalkylated cyclodextrin is sulfobutyl ether-β-cyclodextrin or sulfopropyl ether-β-cyclodextrin.

15. The composition according to claim 2, further comprising a tonicityfier selected from mannitol, sorbitol and xylitol.

16. The composition according to any one of claims 2 and 15, further comprising a pH regulator selected from 2-Amino-2-hydroxymethyl-1,3-propandiol, the acid form or salts of citric acid, acetic acid, histidine, malic acid, phosphoric acid, tartaric acid, succinic acid, MES, HEPES, imidazole, lactic acid, glutaric acid and glycylglycine.

17. The composition according to claim 16, wherein the pH regulator is 2-Amino-2-hydroxymethyl-1,3-propandiol.

18. The composition according to any of the preceding claims, wherein the composition is in the form of a lyophilisate.

19. The composition according to any one of claims 1 to 17, wherein the composition results from the re-constitution of the lyophilisate.

20. The composition according to claim 19, wherein the composition further comprises a solvent selected from aqueous solutions comprising 75-100% of water by volume, preferably 85%-100% by volume, more preferably 90-100% by volume, most preferably 95-100% by volume.

21. A complex between a cyclodextrin and an Epothilone derivative of formula I as defined in claim 2 and wherein R¹,R², R³,R⁴,n, r, p, R^{4a}, R^{4b}, R⁵, R⁶, R⁷, R⁸, G, R⁹, X, R¹⁰, R¹¹, A, R¹² are as defined in claim 2.

22. The complex according to claim 21, wherein the cyclodextrin is β-cyclodextrin or a derivative thereof.

23. The complex according to claim 22, wherein β-cyclodextrin is an alkyl ether β-cyclodextrin, preferably a hydroxy-propyl-β-cyclodextrin and/or a sulfoalkyl ether cyclodextrin.

24. The complex according to claim 23, wherein the sulfoalkylcyclodextrin is sulfobutylether-β-cyclodextrin and the Epothilone is (1S,3S,7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione.

25. The complex according to claim 24, wherein the hydroxyalkylcyclodextrin is hydroxypropyl ether-β-cyclodextrin and the Epothilone is (1S,3S,7S,10R, 11S, 12S, 16R)-7,11-dihydroxy-3-(2-methyl-benzothiazol-5-yl)-10-(prop-2-en-1-yl)-8,8,12,16-tetramethyl-4,17-dioxabicyclo[14,1,0]heptadecane-5,9-dione.

26. A method for treating cancer in a patient comprising administering said patient a therapeutically effective of one or more compound of formula I using the compositions of the present invention, wherein said compositions are administered by intravenous infusion over a period of about 30 minutes in a dose ranging from 10 mg/m² to 35 mg/m².

27. A method according to claim 26, wherein the dose is from 16 mg/m² to 29 mg/m².

28. A method according to claim 26, wherein the dose is 22 mg/m².

29. A method according to anyone of claims 26 to 28, wherein the compositions are administered to the patient every 3 weeks or weekly for 3 weeks followed by one week recovery.

30. A method according to anyone of claims 26 to 28, wherein the compositions are administered to the patient every 3 weeks.

31. A method according to anyone of claims 26 to 28, wherein the compositions are administered to the patient weekly for 3 weeks followed by one week recovery.
